(19) Europäisches Patentamt
European Patent Office
Office européen des brevets

(11) EP 0 531 387 B1

(12) **EUROPEAN PATENT SPECIFICATION**

(45) Date of publication and mention of the grant of the patent:
**14.08.1996 Bulletin 1996/33**

(21) Application number: **91910181.6**

(22) Date of filing: **05.06.1991**

(51) Int. Cl.$^6$: **A61K 7/48**, A61K 7/075, A61K 7/50, A61K 7/00

(86) International application number:
**PCT/FI91/00176**

(87) International publication number:
**WO 91/18588 (12.12.1991 Gazette 1991/28)**

# (54) A METHOD OF REDUCING THE IRRITATING PROPERTIES OF A COSMETIC COMPOSITION

VERFAHREN ZUR VERMINDERUNG DER REIZENDEN EIGENSCHAFTEN VON KOSMETISCHEN ZUSAMMENSETZUNGEN

METHODE PERMETTANT DE REDUIRE LES PROPRIETES IRRITANTES D'UNE COMPOSITION COSMETIQUE

(84) Designated Contracting States:
**AT BE CH DE DK ES FR GB GR IT LI LU NL SE**

(30) Priority: **05.06.1990 US 533684**

(43) Date of publication of application:
**17.03.1993 Bulletin 1993/11**

(73) Proprietor: **Cultor Ltd**
**SF-00241 Helsinki (FI)**

(72) Inventor: **JUTILA, Kirsti Maarit**
**SF-02170 Espoo (FI)**

(74) Representative: **Hansen, Bernd, Dr. Dipl.-Chem. et al**
**Hoffmann, Eitle & Partner**
**Patent- und Rechtsanwälte,**
**Postfach 81 04 20**
**81904 München (DE)**

(56) References cited:
**EP-A- 0 047 916**
**EP-A- 0 186 453**
**EP-A- 0 399 909**
**WO-A-82/02337**
**DE-A- 1 911 144**
**DE-A- 2 750 731**
**DE-A- 3 527 974**
**FR-A- 2 303 536**

- **PATENT ABSTRACTS OF JAPAN, Vol. 7, No. 191, C182, Abstract of JP 58- 92607, publ. 1983-06-02.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 531 387 B1

## Description

### BACKGROUND OF THE INVENTION

The invention refers to a method for reducing the skin-irritating properties of ingredients included in cosmetic compositions.

There is an increasing demand for better and more efficient raw materials for cosmetics and related products. Even the most efficient raw materials can be very irritating or sensitizing for human skin, thus preventing their regular use.

Some of the active compounds in creams, decorative cosmetics, etc., may cause irritation or skin-sensitization. Such active ingredients may include emollients, barrier agents, healing agents, humectants, emulsifiers, preservatives, perfume oils, coloring agents, and/or surfactants, particularly anionic surfactants (e.g., sodium lauryl ether sulphates), and several compounds for skin cleansing (which contain kerosenses, solvents and disinfectants in addition to hard surfactants). Other cosmetics such as deodorants containing ethanol are recognized as having potentially irritating or sensitizing properties. In addition, this problem is often found with many other medicated products.

Cationic compounds such as cetyltrimethylammonium chloride, alkyldimethylbenzylammonium, alkylisoquinidinium, and alkylpyridinium halides are commonly used as hair conditioners. In small amounts, these quaternary ammonium derivatives improve hair manageability.

One drawback of including quaternary ammonium compounds in cosmetics such as shampoos, hair conditioners, creams, lotions, etc. is the fact that great care must be taken when introducing them into commercial products, as most of these materials are known to be skin irritants. More particularly, it is known from the literature that the quaternary compounds of synthetic origin, particularly at higher concentrations (over 10 percent), cause skin irritation and cauterization.

This problem is overcome in hair conditioners and hair rinses because these preparations are diluted about 15 times with water to form the actual rinse used on the hair. Thus, in general, it has been found that when quaternary compounds are present in concentrations upon dilution with water of 0.1 percent, they are usually still safe and effective on the hair shaft.

Certain other cationic compounds introduced into conditioners have been touted as having a lower level of irritancy than earlier compounds. Examples of such include ethoxylated quaternary ammonium phosphates, quaternized fatty acid amino-amides derived from lanolic acids and mink oil, and N-acyl colaminoformylmethylpyridinium derivatives.

Quaternary ammonium salts have also previously been used at concentrations below 0.1 percent as antiseptics in preparations such as aftershave lotions and baby lotions and creams.

One hair treatment composition, described in U.S. Patent No. 4,752,467 (Konrad et al.), contains 0.1-25 percent by weight betaine and 0.1-10 percent by weight of an aliphatic organic acid such as citric acid. The combination of the betaine/aliphatic organic acid is said to provide synergistic conditioning properties which improve the condition of the hair structure, and act as an antioxidant and as a buffer.

DE-Patent No. 1911144 (Medisan Ab) describes a skin-treating composition for treating abnormal skin conditions or as a vehicle for other medicaments, comprising an aqueous phase in which urea and lactic acid are dissolved. The mode of operation of this composition relates to the skin-softening properties of urea and the keratolytic effect of lactic acid. Optional components of this composition include emulsifiers and amino acid derivatives such as betaine.

Other cosmetic formulations also include betaine. Betaine is a general name for an organic tertiary amine with three free organic radicals. Known formulations include betaines in which at least one of the free radicals is a long fatty acid derivative based radical. U.S. Patent No. 4,490,355 (Desai) describes a mixture of cocoamidopropyl betaine and oleomidopropyl betaine which when included in cosmetics is said to improve thickening and foam boosting properties. U.S. Patent No. 4,654,161 (Kollmeier et al.) describes the use of organopolysilaxanes that have betaine groups in hair cosmetics. The use of these siloxane derivatives is said to provide improved compatibility with anionic additives (surfactants) and less irritation than prior organopolysiloxanes with quaternary ammonium groups. EP-Patent No. 286261 (Redken Laboratories) describes the use of zwitterions such as taurines and betaines to increase the substantivity of hydrolysed proteins in keratinous tissues.

It is an object of the present invention to provide a method of reducing the skin irritating properties of a cosmetic composition containing substantially no hydrolysed protein.

### SUMMARY OF THE INVENTION

It has now been surprisingly discovered that adding an effective amount of a quaternary ammonium like compound which does not have any skin irritating properties to cosmetic compositions reduces the skin irritating properties of skin irritating cosmetics and improves their skin compatibility and moisturizing properties. The present invention also relates to the method of reducing the skin irritating properties of a cosmetic composition.

The quaternary ammonium compound, preferably obtained from natural sources, is methanaminium, 1-carboxy-N,N,N-trimethyl-,hydroxide inner salt monohydrate or the corresponding anhydride thereof (also referred to as trimethylglycine or trimethylglycine anhydride respectively).

In the method of the present invention the cosmetic composition preferably comprises one or more cosmetic ingredients selected from the group consisting of an active agent, an emollient, a barrier agent, a healing agent, a humectant, an emulsifier, a preservative, a perfume oil, a coloring agent, a deodorizing agent, a surfactant, a solvent and mixtures of the above and one or more of the cosmetic ingredient having irritating properties when applied to human skin, and trimethylglycine or anhydride in an amount effective to substantially neutralize the skin-irritating properties of the cosmetic ingredient.

The dosage of trimethylglycine (or its anhydride) is from 0.1 to 50, preferably 1 - 15, percent by weight.

The cosmetic compositions as used in the method of the present invention preferably include skin cleansers, body shampoos, hair conditioners, skin creams, lotions or deodorants.

DETAILED DESCRIPTION

The addition of trimethylglycine, a natural quaternary ammonium like compound methanaminium, 1-carboxy-N,N,N-trimethyl-,hydroxide inner salt monohydrate or the corresponding anhydride (trimethylglycine/ah)

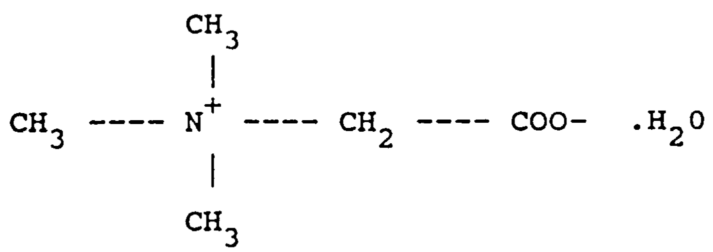

has now surprisingly been found to reduce the skin irritation properties or sensitisation reactions of cosmetic compounds including irritating ingredients such as sodium lauryl ether sulphate, and other anionic surfactants, cetyltrimethylammonium chloride, alkyldimethylbenzylammonium, and alkylisoquinidinium, alkylpyridinium halides or solvents. Trimethylglycine (or anhydride thereof) in question has been found to improve the skin compatibility and moisturizing characteristics of cosmetic compositions. Problems mentioned above are often found in products containing irritants, including skin and hand cleaners, skin lotions, skin creams, as well as medicated products. The dosage of methanaminium, 1-carboxy-N,N,N-trimethyl-, hydroxide, inner salt monohydrate or corresponding anhydride is between 0,1 and 50%, preferably 1 - 15 w/w.

The invention is further elucidated by the following examples.

Examples 1-16

The effect of methanaminium, 1-carboxy-N,N,N-trimethyl,-hydroxide, inner salt monohydrate on skin compatibility and irritation were tested by human patch testing.

The patch testing was conducted as follows. In each of these Examples, the test materials were applied on the upper outer arm and the application arm was occluded with a patch for an initial contact period of 24 hours. The patches were then removed and the skin reaction was assessed using a numerical scoring system one hour later.

In order to assess the degree of irritancy, several signs symptomatic of skin irritation and their severity were noted using the scoring system below:

0 = no visible relevant reaction
1 = reaction just present
2 = slight reaction
3 = moderate reaction
4 = severe reaction

The signs of irritation were awarded different ratings of significance as follows:

| SIGN | ABBREVIATION | RATING |
|---|---|---|
| Vesicles | V | 5 |
| Odema | Oe | 4 |
| Erythema | R | 3 |
| Flakiness | F | 2 |
| Dryness | D | 1 |
| wrinkling | W | 1 |
| Glazing | G | 1 |

In order to obtain a numerical value for the total reaction at each site the severity score for each sign was multiplied by the rating. The resulting values were summed to give a total score for the degree of irritation at that site.

For example, the total score for a site assessed as R3, D2 and Gl would be:

$$(3 X 3) + (2 X 1) + (1 X 1) = 12$$

The skin irritation scores for the test and control products were compared statistically using the binomial probability test.

Immediately after assessment an identical fresh patch was applied to the same skin site for a further 24 hours (for a total of 48 hours) and skin reactions were again examined one hour after removal. The materials tested and the mean skin irritation scores after 24 hours and after 48 hours were as follows:

| Example | Material Tested | Skin Irritation | |
|---|---|---|---|
| | | 24 hr. | 48 hr. |
| 1 | Deionized water (control) | 1.28 | 0.96 |
| 2 | Trimethylglycine 50% w/v in deionized water | 0.44 | 0.32 |
| 3 | sodium lauryl ether sulfate (SLES) 10.3% | 1.76 | 4.28 |
| 4 | SLES 10.3% + trimethylglycine 7% | 0.56 | 0.80 |
| 5 | SLES 10.3% + trimethylglycine 5% | 0.72 | 1.92 |
| 6 | SLES 10.3% + trimethylglycine 3.5% | 0.76 | 2.40 |
| 7 | SLES 10.3% + trimethylglycine 2% | 1.12 | 2.92 |
| 8 | SLES 5.15% + trimethylglycine 3.5% | 0.88 | 2.88 |
| 9 | Deodorant (Control) | 0.96 | -- |
| 10 | Deodorant + 5% trimethylglycine | 0.65 | -- |
| 11 | Medical cleaner | 1.50 | 2.12 |
| 12 | Medical cleaner + 5% trimethylglycine | 1.27 | 1.50 |
| 13 | Eau de cologne | -- | -- |
| 14 | Eau de cologne + 5% trimethylglycine | -- | -- |
| 15 | After Shave Lotion (Control) | 1.46 | -- |
| 16 | After Shave Lotion + 5% trimethylglycine | 0.85 | -- |

It can be seen from the foregoing numerical results that trimethylglycine significantly reduced the irritancy of the sample to the skin. Where a "--" is indicated in the chart above numerical results were not obtained.

Example 17

Transepidermal Water Loss (TEWL)

Irritants have much stronger effect on dry skin; thus ingredients of cosmetic compounds should not cause any increasing normal TEWL.

The effect of methanaminium, 1-carboxy-N,N,N-trimethyl-,hydroxide inner salt monohydrate on moisturizing and skin smoothness was tested by the transepidermal water loss from skin (TEWL).

In example 17 a leading marketed skin cream was applied under an occlusive patch.

Skin Care Cream (commercially marketed)

| Ingredients | |
|---|---|
| Glycerol monostearate | 2.5 |
| Cetyl stearyl ethoxylate | 5.0 |
| Emulsifier (Stearic acid) | 2.0 |
| White oil | 25.0 |
| Glycerol | 10.0 |
| Triethanolamine | 0.8 |
| Water | Balance |

The same cream containing 50% w/w trimethylglycine was applied under an occlusive patch at a different skin site. As a control, blank occlusive patches were also applied to a third skin site. The transepidermal water loss from skin (TEWL) was measured.

The test subjects were twelve female volunteers between 18 and 65 years of age. Panelists were selected on the + criteria that they had normal healthy skin on their forearms and were able to provide stable TEWL values.

Three sites were marked on the volar forearm of each volunteer at approximately equal intervals between the elbow crease and the wrist. The subject was then seated comfortably with her forearms exposed for a period of half an hour so that they would equilibrate with conditions of temperature and relative humidity in the laboratory.

TEWL readings were taken from each skin site to provide normal untreated water loss levels. Treatment was made via 12 mm occlusive Finn chambers. A blank Finn chamber served as the control site. The lotion samples were applied by immersing Whatman 3MM filter paper discs in the sample, removing excess product against the edge of the container and placing the disc in the Finn chamber using forceps. Each chamber was applied to one of the marked sites on the forearm and held in position with Scanpor non-occulsive surgical tape. The pattern of application of treatments to sites was randomized and recorded.

18 to 24 hours later each subject returned and was re-equilibrated as above. The chambers were then removed and the skin was blotted with tissue to absorb surface moisture. TEWL readings were recorded at 1 minute intervals from the time of removal of treatment until a steady valve was obtained, a period of ten to twenty-five minutes depending on the individual concerned and the treatment applied.

Temperature and relative humidity were recorded at the start of each TEWL reading as gross changes can significantly alter the rate of water loss. The results for each panelist are presented graphically below. An analysis of the results revealed the following:

1) The 1 minute TEWL reading immediately after patch removal is lower for the cream containing trimethylglycine than for the cream alone indicating that the trimethylglycine-treated sites lost water less rapidly. The blank patch control is lower still and represents the skin hydrating effect of occlusion, without adding additional moisture to the skin.

2) The final TEWL value obtained when readings approach a constant value is lower for the cream containing trimethylglycine than for the cream alone, and frequently also the blank control, again indicating better moisture retention at trimethylglycine-treated skin sites.

3) The rate at which the TEWL value decreases towards the normal untreated level is in general more rapid for the cream containing trimethylglycine than for the cream alone or the blank control.

These results are summarized in the table below:

| Initial TEWL level ($g/m^2h$) | | | | Final TEWL level ($g/m^2h$) | | | Time for 75 % reduction in TEWL rate (min) | | |
|---|---|---|---|---|---|---|---|---|---|
| Panel No. | Blank | Cream | Cream +TMG | Blank | Cream | Cream +TMG | Blank | Cream | Cream +TMG |
| 1 | * | 58 | 40 | 7 | 8 | 5 | * | 7 | 5 |
| 2 | 36 | 50 | 48 | 10 | 12 | 8 | Not reached | 14 | 7 |
| 3 | 34 | 41 | 54 | 8 | 9 | 7 | 16 | 12 | 6 |
| 4 | 20 | 41 | * | 7 | 12 | 8 | Not reached | Not reached | * |
| 5 | 30 | 63 | 39 | 8 | 18 | 8 | Not reached | Not reached | 13 |
| 6 | 17 | 61 | 53 | 9 | 20 | 8 | Not reached | 13 | 8 |
| 7 | 17 | 78 | 45 | 7 | 22 | 3 | Not reached | Not reached | 5 |
| 8 | 26 | 71 | 65 | 11 | 26 | 11 | Not reached | Not reached | 7 |
| 9 | 25 | 74 | 63 | 6 | 19 | 8 | 11 | Not reached | 7 |
| 10 | 18 | i | 34 | 7 | i | 6 | Not reached | i | 7 |
| 11 | 23 | 53 | 53 | 10 | 17 | 9 | Not reached | Not reached | 8 |
| 12 | 26 | 54 | 30 | 6 | 13 | 4 | 17 | 18 | 7 |
| The "*" indicates that the evapormeter probe shifted over in the early part of curve so readings were not valid. The "i" indicates that the patch came loose over the application period. | | | | | | | | | |

It can be seen that under these test conditions there were clear indications that the addition of trimethylglycine to the skin cream resulted in improved moisture retention when the product was applied to the skin.

Example 18

Example 18 provides illustrative formulations including ingredient with irritating properties for a skin cream, body shampoo and skin cleanser in accordance with the invention.

Skin cream

| Ingredient | Amount (%) |
|---|---|
| Trioleyl phosphate | 3.0 |
| Petrolatum | 18.0 |
| Glyceryl stearate | 5.0 |
| Isopropyl palmitate | 4.0 |
| Cetyl alcohol | 2.0 |
| Stearyl heptanoate (irr.) | 0.5 |
| Cetearyl octanoate (irr.) | 0.5 |
| Sorbitol | 5.0 |
| Trimethylglycine | 5.0 |
| Water, perfume, preservative (irr.) | q.s. |

Shampoo

| Ingredient | Amount (%) |
|---|---|
| Sodium lauryl ether sulphate 30% (irr.) | 40.0 |
| Coconut monoethanolamide | 2.0 |
| Trimethylglycine | 5.0 |
| Perfume, colour, water | q.s. |
| HCl/NaOH | q.s. to pH 6.5-7.2 |

Sodium lauryl ether sulphate is known to have a hard cleansing effect; thus because of the side effect (e.g. too effective fat removal from the skin and hair) it cannot be used as such in shampoos. The addition of trimethylglycine remarkably reduces the irritation effect.

Skin cleanser for oily skin

| Ingredient | Amount (%) |
|---|---|
| Triclosan (deodorising agent; irr.) | 3.0 |
| Menthol (irr.) | 10.0 |
| DEA-oleth-3 phosphate | 2.5 |
| Hydroxypropylcellulose | 2.5 |
| Amphoteric-1 | 5.0 |
| Trimethylglycine | 5.0 |
| Water | 37.0 |
| Ethanol(96%) | 35.0 |

Trimethyl glycine reduces the irritating properties of irritants.

Example 19 - Smoothness Test

Body Lotion With Improved Skin Compatibility

| Ingredient | Amount (%) |
|---|---|
| Emulsifier | 2.0 |
| Cetylalcohol (irr.) | 2.5 |
| Isopropylstearate | 4.5 |
| Preservative (potential irr.) | 0.03 |
| Water | 78.77 |
| 1,2-Propyleneglycol | 6.0 |
| Trimethylglycine | 0 or 6.0 |
| Parfum oil | 0.2 |

The effect of methanaminium, 1-carboxy-N,N,N-trimethyl-,hydroxide inner salt monohydrate on skin tested by the Zeiss-spektrophotometer. This formula was tested by using ten female volunteers. The body lotion was used for 8 days. The smoothness of the skin was measured by a Zeiss-spektrophotometer by 660 nm. The results show that the addition of trimethylglycine moderately improves the smoothness of the skin.

Smoothness of skin was calculated by the following equation:

$$= \frac{\text{extinction after treatment}}{\text{initial extinction}} \text{ X 100}$$

Results for the ten subjects are reported below:

| Age of the volunteer | 0% | 6% Trimethylglycine |
|---|---|---|
| 43 | 99.8 | 91.8 |
| 44 | 99.3 | 89.4 |
| 51 | 75.9 | 59.4 |
| 50 | 98.6 | 79.3 |
| 70 | 97.5 | 89.4 |
| 47 | 98.3 | 91.9 |
| 34 | 100.3 | 97.1 |
| 64 | 75.4 | 97.8 |
| 30 | 100.0 | 97.4 |
| 51 | 95.3 | 96.5 |
| Average | 94.0 | 89.0 |

Example 20 - Moisture Holding Capacity Using Corneometer

The same body lotion formula used in Example 19 was used in tests where the moisturizing effect of trimethylglycine was measured by a Corneometer. The moisture holding capacity in the skin was increased when the trimethylglycine level in the product increased from 0% to 2% to 6%.

The same group of test persons and model products were used as in Example 19. Medial part of skin of arms were used (5 x 2.5 $cm^2$). Different test areas for different model products were used. The instrument used for measurement of the lotions, skin moisturizing effect was a Corneometer Combi (Courage & Khazaka Electronic GmbH, FRG). The subjects were acclimatized in a controlled atmosphere (vH 60 %, T° 24°C) for 30 minutes before 0-values of different skin areas were measured. Body lotions were used twice daily (12 hr. intervals). Duration of the trials was 8 days.

The following are hydration measurements made by the Corneometer Combi:

| Product | Measurement | | | | | | | | |
|---|---|---|---|---|---|---|---|---|---|
| | | after Appl. | Minutes | | | | | | |
| | | | 10 | 20 | 30 | 40 | 60 | 90 | 120 |
| Control | 107.8 | 106.3 | 106.8 | 107.8 | 107.7 | 108.7 | 107.6 | 108.2 | 107.8 |
| 0 % | 104.5 | 155.8 | 118.5 | 118.4 | 116.1 | 115.7 | 113.2 | 112.6 | 111.8 |
| 2 % | 104.0 | 152.9 | 120.0 | 119.2 | 115.9 | 117.1 | 113.8 | 113.8 | 112.8 |
| 6 % trimethylglycine | 103.6 | 150.4 | 122.1 | 118.7 | 116.1 | 117.6 | 116.1 | 116.3 | 114.9 |

## Claims

1. A method of reducing the skin irritating properties of a cosmetic composition containing substantially no hydrolyzed protein, characterized in that methanaminium, 1-carboxy-N,N,N-trimethyl-, hydroxide, inner salt monohydrate (trimethyl glycine) having the chemical structure:

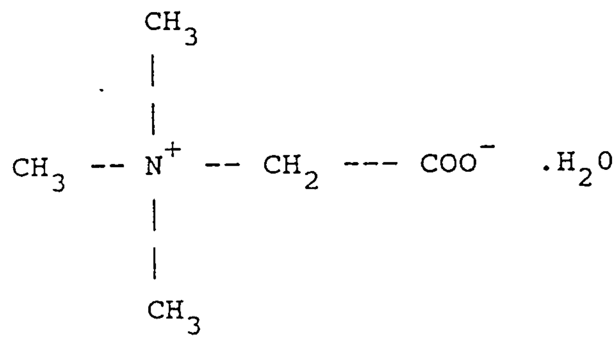

or an anhydride thereof is incorporated into the cosmetic composition in an amount effective to neutralize the skin-irritating properties of the cosmetic composition.

2. The method according to claim 1, characterized in that the methanaminium, 1-carboxy-N,N,N-trimethyl-, hydroxide, inner salt is added in a concentration of 0,1 - 50%, preferably 0,1 - 15% by weight.

3. The method according to claim 1 or 2, characterized in that the cosmetic composition is a skin cleanser, a skin cream, or body shampoo.

4. The method according to any of the claims 1 to 3, characterized in that the cosmetic composition is a cream, ointment, emulsion, or an aerosol.

5. The method according to any of the claims 1 to 4, characterized in that the cosmetic composition includes a deodorant.

6. The method according to any of the claims 1 to 5, characterized in that the cosmetic composition comprises:

   (a) one or more cosmetic ingredients selected from the group consisting of an active agent, an emollient, a barrier agent, a healing agent, a humectant, an emulsifier, a preservative, a perfume oil, a coloring agent, a deodorising agent, a surfactant, a solvent and mixtures of the above; wherein one or more of the above cosmetic ingredients have irritating properties when applied to human skin; and

   (b) trimethylglycine or anhydride thereof in an amount effective to neutralize the skin-irritating effects of said cosmetic ingredient(s).

## Patentansprüche

1. Verfahren zur Herabsetzung von die Haut reizenden Eigenschaften einer kosmetischen Zusammensetzung, die im wesentlichen kein hydrolysiertes Protein enthält,
   dadurch **gekennzeichnet,**
   daß man Methanaminium-1-carboxy-N,N,N-trimethyl-Hydroxid, Innersalz-Monohydrat, (Trimethylglycin) der chemischen Struktur:

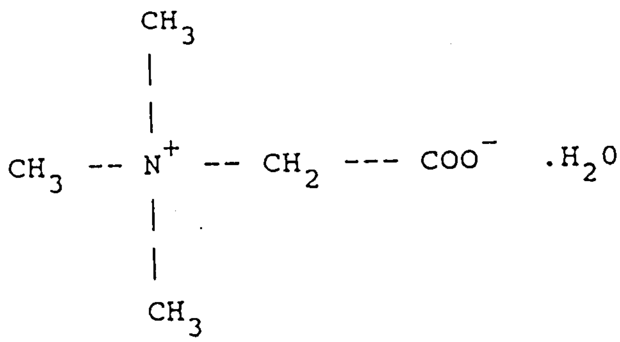

oder ein Anhydrid davon in die kosmetische Zusammensetzung in einer wirksamen Menge einbringt, um die die Haut reizenden Eigenschaften der kosmetischen Zusammensetzung zu neutralisieren.

**2.** Verfahren gemäß Anspruch 1,
dadurch **gekennzeichnet,**
daß das Methanaminium-1-carboxy-N,N,N-trimethyl-Hydroxid, inneres Salz, in einer Konzentration von 0,1 bis 50, vorzugsweise von 0,1 bis 15, Gew.-% zugefügt wird.

**3.** Verfahren gemäß Anspruch 1 oder 2,
dadurch **gekennzeichnet,**
daß die kosmetische Zusammensetzung ein Haut-Reiniger, eine Haut-Creme oder ein Körper-Shampoo ist.

**4.** Verfahren gemäß einem der Ansprüche 1 bis 3,
dadurch **gekennzeichnet,**
daß die kosmetische Zusammensetzung eine Creme, Salbe, Emulsion oder ein Aerosol ist.

**5.** Verfahren gemäß einem der Ansprüche 1 bis 4,
dadurch **gekennzeichnet,**
daß die kosmetische Zusammensetzung ein Deodoriermittel enthält.

**6.** Verfahren gemäß einem der Ansprüche 1 bis 5,
dadurch **gekennzeichnet,**
daß die kosmetische Zusammensetzung umfaßt:

(a) einen oder mehrere kosmetische Bestandteile, ausgewählt aus der Gruppe, bestehend aus einem Wirkstoffmittel, Weichmacher, Barrieremittel, Heilungsmittel, Feuchtigkeitsmittel, Emulgator, Konservierungsstoff, Parfümöl, Färbungsmittel, Deodoriermittel, oberflächenaktiven Mittel, Lösungsmittel und aus Mischungen davon, worin einer oder mehrere der obigen kosmetischen Bestandteile bei Aufbringung auf die menschliche Haut Reizungseigenschaften aufweisen, und

(b) Trimethylglycin oder das Anhydrid davon in einer zum Neutralisieren der die Haut reizenden Wirkungen der genannten kosmetischen Bestandteile wirksamen Menge.

**Revendications**

**1.** Procédé pour réduire les propriétés d'irritation de la peau d'une composition cosmétique ne contenant pratiquement aucune protéine hydrolysée, caractérisé en ce que du sel interne monohydraté de l'hydroxyde de 1-carboxy-N,N,N-triméthyl méthanaminium, (triméthylglycine) ayant la structure chimique :

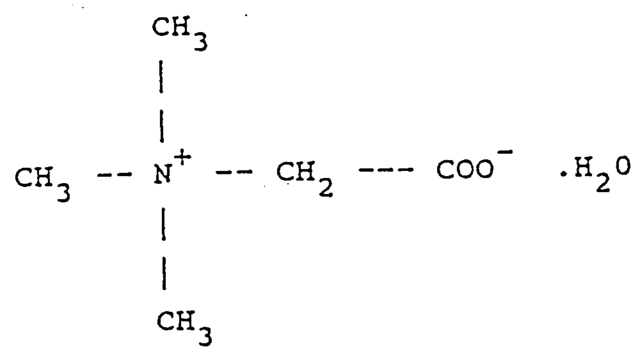

ou un de ses anhydrides est incorporé dans la composition cosmétique dans une quantité efficace pour neutraliser les propriétés d'irritation de la peau de la composition cosmétique.

2. Procédé selon la revendication 1, caractérisé en ce que le sel interne monohydraté de l'hydroxyde de 1-carboxy-N,N,N-triméthyl méthanaminium, est ajouté dans une concentration de 0,1 - 50 %, de préférence de 0,1 à 15 %, en poids.

3. Procédé selon la revendication 1 ou 2, caractérisé en ce que la composition cosmétique est un produit de nettoyage de la peau, une crème pour la peau, ou un shampooing pour le corps.

4. Procédé selon l'une quelconque des revendications 1 à 3, caractérisé en ce que la composition cosmétique est une crème, une pommade, une émulsion ou un aérosol.

5. Procédé selon l'une quelconque des revendications 1 à 4, caractérisé en ce que la composition cosmétique comprend un déodorant.

6. Procédé selon l'une quelconque des revendications 1 à 5, caractérisé en ce que la composition cosmétique comprend :

(a) un ou plusieurs ingrédients cosmétiques choisis dans le groupe constitué d'un agent actif, d'un émollient, d'un agent formant barrière, d'un agent de cicatrisation, d'un agent humidifiant, d'un émulsionnant, d'un agent de conservation, d'une huile parfumée, d'un agent colorant, d'un agent déodorisant, d'un agent tensio-actif, d'un solvant et des mélanges de ceux-ci; où un ou plusieurs des ingrédients cosmétiques ci-dessus présentent des propriétés d'irritation lorsqu'ils sont appliqués à la peau humaine; et
(b) de la triméthylglycine ou un anhydride de celle-ci dans une quantité efficace pour neutraliser les effets d'irritation de la peau desdits ingrédients cosmétiques.